# EUROPEAN PATENT SPECIFICATION

(11) **EP 4 384 127 B1**
(45) Date of publication and mention of the grant of the patent: **23.09.2026**
(21) Application number: 22764510.8
(22) Date of filing: 08.08.2022
(51) Int. Cl.: A61F 5/451, A61F 5/44

(54) **PORTABLE URINE COLLECTION SYSTEM**
TRAGBARES URINSAMMELSYSTEM
SYSTÈME PORTATIF DE COLLECTE D'URINE

(30) Priority: 09.08.2021 US 202163230894 P
(43) Date of publication of application: 19.06.2024
(73) Proprietor: Purewick Corporation, El Cajon, California 92020 (US)
(72) Inventor: LAI, Kuilin, Watkinsville, Georgia 30677 (US); PATEL, Puja, Lawrenceville, Georgia 30044 (US); JAGANNATHAN, Sudhakar, Alpharetta, Georgia 30004 (US); GORDON, Tacarra, Loganville, Georgia 30052 (US); JARDINE, Nicholas, Holly Springs, North Carolina 27540 (US); RICHARDSON, Tamika, Schenectady, New York 12306 (US); GARVEY, Tony, Listowel, V31CK75 (IE); FOGARTY, Padraig, Tipperary, V94K5P5 (IE); CARACCIOLA, Meghan, Washington, District of Columbia 20003 (US)
(74) Representative: Hoffmann Eitle
(86) International application number: PCT/US2022/039711
(87) International publication number: WO 2023/018656

(56) References cited:
- WO-A1-00/25651
- WO-A1-2022/150463
- US-A- 4 631 061
- US-A1- 2002 087 131
- US-A1- 2007 225 668

## Description

### CROSS-REFERENCE TO RELATED APPLICATIONS

### BACKGROUND

An individual may have limited or impaired mobility such that typical urination processes are challenging or impossible. For example, the individual may have surgery or a disability that impairs mobility. In another example, the individual may have restricted travel conditions such as those experienced by pilots, drivers, and workers in hazardous areas. Additionally, fluid collection from the individual may be needed for monitoring purposes or clinical testing.

Bed pans and urinary catheters, such as a Foley catheter, may be used to address some of these circumstances. However, bedpans and urinary catheters have several problems associated therewith. For example, bedpans may be prone to discomfort, spills, and other hygiene issues. Urinary catheters be may be uncomfortable, painful, and may cause urinary tract infections. Conventional fluid collection devices also may be limited to use when a patient is confined to a bed in a supine position.

US2007/0225668 A1 discloses mounting on a wheelchair or electrically-powered scooter a self-contained and battery-powered urine collection system using a pump, such as a peristaltic pump. Suggested is to use the power supply of the electric scooter to re-charge the batteries of the urine collection system. Users and manufacturers of fluid collection devices continue to seek new and improved devices, systems, and methods to collect fluid.

### SUMMARY

The invention is defined in claim 1 below and the dependent claims are directed to optional features and preferred embodiments of the invention.

Features from any of the embodiments of the invention disclosed below may be used in combination with one another, provided that the combination falls under the scope of the invention as defined by the claims. In addition, other features and advantages of the present invention will become apparent to those of ordinary skill in the art through consideration of the following detailed description and the accompanying drawings.

### BRIEF DESCRIPTION OF THE DRAWINGS

The drawings illustrate several embodiments of the present invention, wherein identical reference numerals refer to identical or similar elements or features in different views or embodiments shown in the drawings.
**FIG. 1** is a block diagram of a portable fluid collection system.
**FIG. 2A** is an isometric view of a fluid collection system.
**FIG. 2B** is an isometric view of a portable fluid collection system.
**FIG. 3** is an isometric view of a fluid collection container.
**FIG. 4A** is a rear view of a portable carrying case for a fluid collection system.
**FIG. 4B** is a cross sectional view of a portable fluid collection system and carrying case.
**FIG. 5A** is a rear isometric view of a fluid collection system including an alternator coupled to a wheelchair.
**FIG. 5B** is a front isometric view of a fluid collection system including a pump coupled to a wheelchair.
**FIG. 5C** is a schematic view of a pump.
**FIG. 6** is a schematic view of a filter system for a fluid collection system.
**FIG. 7** is a flow diagram of a method for assembling a portable fluid collection system.

### DETAILED DESCRIPTION

The disclosure which follows relates to fluid collection systems and related methods. Many users of fluid collection devices are over 65 years old with limited mobility, often relying on wheelchairs as a primary mode of transportation. Many users also spend a significant amount of their day in a seated or supine position. Users and caregivers, then, are benefited from a fluid collection system that may be both discreet and mobile, allowing users to use the fluid collection system to collect fluid both at home and on the go.

As described below, a fluid collection system may be configured to be capable for extended use at home or may be relatively compact and configured to be portable. Embodiments of the fluid collection systems described herein are both able to include large capacity of urine collection for at-home (e.g., overnight) use and mobile and discreet, allowing a user to participate in social activities without alerting others to the incontinence of the users. For example, the fluid collection systems may include a portable carrying case configured to detachably couple to a wheelchair and hold at least a pump of a fluid collection system therein to obscure the pump from view and/or dampen noise outside the fluid collection system. The fluid collected in the fluid collection systems described herein also may be stored in a fluid collection container that is obscured by the portable carrying case from view and/or obscures the fluid held in the fluid collection container. Portable can be defined as able to be easily carried or moved, especially because being of a lighter and smaller version than fluid collection systems that include large capacity of urine collection. In other words, the portable carrying case can be carryable by a human, such as a caretaker or a patient.

The fluid collection system also includes a fluid collection device configured to be positioned at least proximate to a urethra of a user and may include also a first tube in fluid communication with the fluid collection device where the pump may be configured to pull an at least partial vacuum on an interior region of the fluid collection container effective to draw fluid from the fluid collection device through the first tube into the fluid collection container.

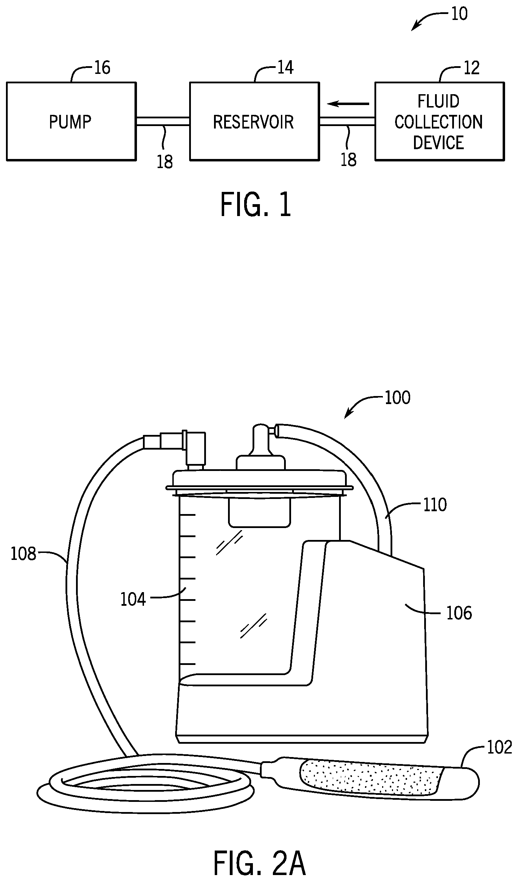

FIG. 1 is a block diagram of a fluid collection system 10. The fluid collection system 10 may be included in embodiments of fluid collection systems described herein. The system 10 includes a fluid *(e.g.,* urine) collection device 12 *(e.g.,* any of the fluid collection assemblies disclosed herein), a fluid collection container 14 (or reservoir), and a peristaltic pump 16 (portable vacuum device). The fluid collection device 10, the fluid collection container 14, and the pump 16 may be fluidly coupled to each other via one or more tubes 18. For example, fluid collection device 10 may be operably coupled to one or more of the fluid collection container 14 or the pump 16 via the tube 18. In some embodiments, the pump 16 may be coupled directly to the fluid collection container 14. Fluid (e.g., urine or other bodily fluids) collected in the fluid collection device 10 may be removed from the fluid collection device 10 via the tube 18 coupled to the fluid collection device 12. Suction force may be introduced into the chamber of the fluid collection device 12 via the inlet of the tube 18 responsive to suction (e.g., vacuum) force applied at the outlet of the tube 18.

The suction force may be applied to the outlet of the tube 18 by the pump 16 either directly or indirectly. The suction force may be applied indirectly via the fluid collection container 14. For example, the outlet of the tube 18 may be disposed within or fluidly coupled to an interior region of the fluid collection container 14 and an additional tube 18 may extend from the fluid collection container 14 to the pump 16. Accordingly, the pump 16 may apply suction to the fluid collection device 12 via the fluid collection container 14. The suction force may be applied directly via the pump 16. For example, the outlet of the tube 18 may be disposed within the pump 16. An additional tube 18 may extend from the pump 16 to a point outside of the fluid collection device 12, such as to the fluid collection container 14. In such examples, the pump 16 may be disposed between the fluid collection device 12 and the fluid collection container 14.

The fluid collection container 14 is sized and shaped to retain a fluid therein. The fluid collection container 14 may include a bag (e.g., drainage bag), a bottle or cup (e.g., collection jar), or any other enclosed container for storing bodily fluid(s) such as fluid. In some examples, the tube 18 may extend from the fluid collection device 12 and attach to the fluid collection container 14 at a first point therein. An additional tube 18 may attach to the fluid collection container 14 at a second point thereon and may extend and attach to the pump 16. Accordingly, a vacuum (e.g., suction) may be drawn through fluid collection device 12 via the fluid collection container 14. Fluid, such as urine, may be drained from the fluid collection device 12 using the pump 16.

The pump 16 vacuum source may include one or more of a manual vacuum pump, and electric vacuum pump, a diaphragm pump, a centrifugal pump, a displacement pump, a magnetically driven pump, or any pump configured to produce a vacuum. The pump 16 may provide a vacuum or suction to remove fluid from the fluid collection device 12. In some examples, the pump 16 may be powered by one or more of a power cord (*e.g.,* connected to a power socket), an alternator, one or more batteries, or even manual power (*e.g.,* a hand operated vacuum pump). The vacuum sources disclosed herein may include one or more of a switch, a button, a plug, a remote, or any other device suitable to activate the pump 16.

Many embodiments of fluid collection systems described herein are configured to be worn by a user, positioned on a surface such as a table, and/or securable or mountable to a wheelchair. **FIGS. 2A-2B** is an isometric view of a fluid collection system 100. The fluid collection system 100 may include a fluid collection device 102 configured to receive fluid (e.g. urine) from a user. The fluid collection assembly 102 may be removably attached to a fluid collection container 104 of the fluid collection system 100. The fluid collection container 104 may be integrated into a housing 106 that may also include a pump therein. The fluid collection system may further include a collection tube 108 and a suction tube 110. The collection tube 108 may be disposed between the fluid collection device 102 and the fluid collection container 104. The collection tube 108 may fluidly connect the internal volume of the fluid collection container 104 with the fluid collection assembly 102. The suction tube 110 may couple the pump suction and/or discharge to the fluid collection container 104. The pump disposed within the housing 106 may be in fluid communication with the internal volume of the fluid collection container 104 via the suction tube 110. The housing 106 may also be configured to store or house internal electrical and mechanical components. For example, the housing may include a pump (not shown in FIG. 2A) configured to modulate pressure within the container 104.

The fluid collection device 102 may be positioned at least proximate to a urethral opening. The fluid collection device shown in **FIGS. 2A-2B** is an example of a female fluid collection assemblies configured to collect fluid(s) from females (e.g., collect urine from a female urethra). Further examples of female fluid collection assemblies are disclosed in U.S. Patent No. 10,390,989 issued on August 27, 2019. However, the fluid collection assemblies, systems, and methods disclosed herein may include male fluid collection assemblies and/or devices shaped, sized, and otherwise configured to collect fluid(s) from males (e.g., collect urine from a male urethra).

In some embodiments, the collection tube 108, the suction tube 110 and/or other tubing of the fluid collection system 100 may include a flexible material such as materials tubing (*e.g.*, medical tubing). Such material tubing may include a thermoplastic elastomer, polyvinyl chloride, ethylene vinyl acetate, polytetrafluoroethylene, flexible metal, ceramic and composite material tubing etc. The collection tube 108 may include silicon or latex. In some embodiments, the collection tube 108 may be constructed of any suitable material to be impermeable to fluids such that fluids may be drawn from the fluid collection assembly 102 and into the collection tube 108. In some embodiments, the collection tube 108 may include one or more portions that are resilient, such as by having one or more of a diameter or wall thickness that allows the collection tube 108 to be flexible.

The fluid collection container 104 may be integrated into the housing 106. In some embodiments, the container 104 may include at least an inlet coupled to the collection tube 108, an outlet coupled to a drain tube and a vacuum port coupled to the pump. In some embodiments, the container 104 may be opaque or clear according to different embodiments and may include a generally rectangular front or rear profile as shown in **FIGS. 2A-2B****.** As shown in **FIG. 2A****,** in some embodiments, the fluid collection container 104 may include a large capacity from about 1000mL to about 2000mL. The fluid collection container 104 may include the large capacity for home use, where the fluid collection system 100 may be used for a long duration, without needing to refill and/or exchange the fluid collection container 104. Uses for the large capacity fluid collection container 104 may include travelling long distances, overnight use, and times when a caregiver may not be able to attend the user and/or empty/replace the fluid collection container 104.

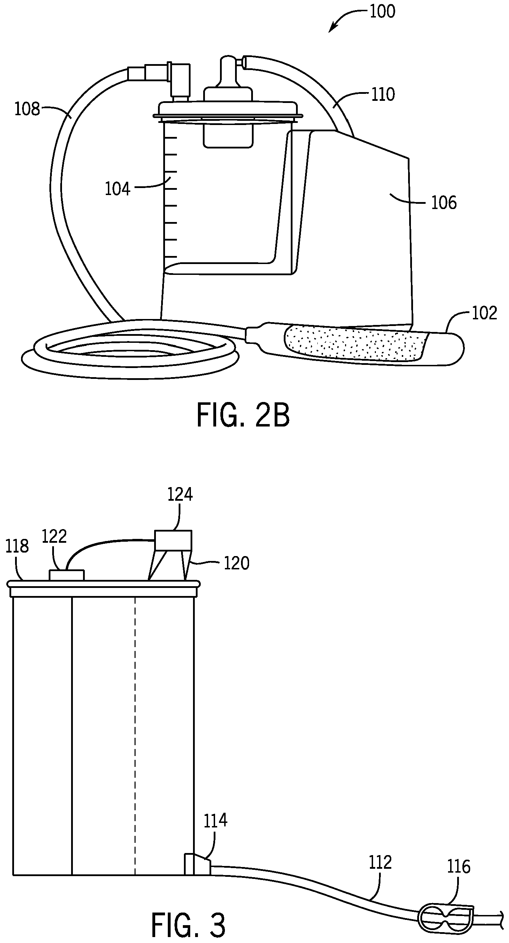

As shown in **FIG. 2B****,** in some embodiments, the fluid collection container 104 may include a relatively smaller capacity from about 100mL to about 500mL. Uses for the smaller capacity fluid collection container 104 may include portability, discretion, less weight and more convenient size of the fluid collection system 100, and improved independence for the user. The fluid collection container 104 may be rectangular shaped. The rectangular shape may improve the portability of the fluid collection system 100 by making the system easier to put into a portable carrying case (*e.g.* a backpack). In some embodiments, the fluid collection container 104 may be reusable. The user and/or caretaker may empty, clean, and replace the fluid collection container 104. In other embodiments, the fluid collection container 104 may be disposable. The fluid collection container 104 may be thrown away and a replacement container installed.

**FIG. 3** illustrates an embodiment of fluid collection container 104. The fluid collection container 104 may include a durable plastic canister and may include a capacity from about 1000mL to about 2000mL or from about 100mL to about 500mL. The fluid collection container 104 may include a rectangular shape to allow the container 104 to securely and discretely fit within a portable carrying case. The fluid collection container 104 may be reusable and dishwasher safe, and may include a generally rigid material such as polycarbonate, plastic, rubber, metal, glass, combinations thereof, or any other suitable materials. In other embodiments, the fluid collection container 104 may be disposable and biodegradable.

In some embodiments, the fluid collection container 104 may include a drain tube 112 to remove fluid from the fluid collection container 104. Fluid collected in the fluid collection container 104 may be emptied through the drain tube 112. The drain tube 112 may be coupled to a drain port 114. In some embodiments, the drain port 114 may be located in a bottom corner of the fluid collection container 104. In some embodiments, the drain port 114 may automatically open when the drain tube 112 couples to the drain port 114. The drain port 114 may include a valve that opens when the drain tube 112 is coupled to the drain port 114. For example, the drain port 114 may include a split septum device that opens when the drain tube 112 is inserted. In other embodiments, the drain port 114 may be a one-way valve that is activated open when the drain tube 112 is connected.

In some embodiments, the drain tube 112 may include a valve 116 that may be opened to empty the fluid collection container 104 directly or a pump may be coupled to the fluid collection container 104 and configured to apply a pressure to the fluid for more rapid disposal. The valve 116 may include a clamp, a ball valve, a butterfly valve, or any suitable valve.

In some embodiments, the fluid collection container 104 includes a lid 118 and a drain spout 120 and vent spout 122 coupled to the lid 118. In some embodiments, the lid 118 further includes connectors (not shown) for the collection tube 108 and the suction tube 110. The drain spout 120 may include a cap 124 that covers the drain spout 120 when not in use. The cap 124 may be connected to a cover (not shown) for the vent spout 122 and configured such that the cap 124 is removed and the vent spout 122 is open when the fluid is dispensed, to aid in proper air flow as the fluid is removed.

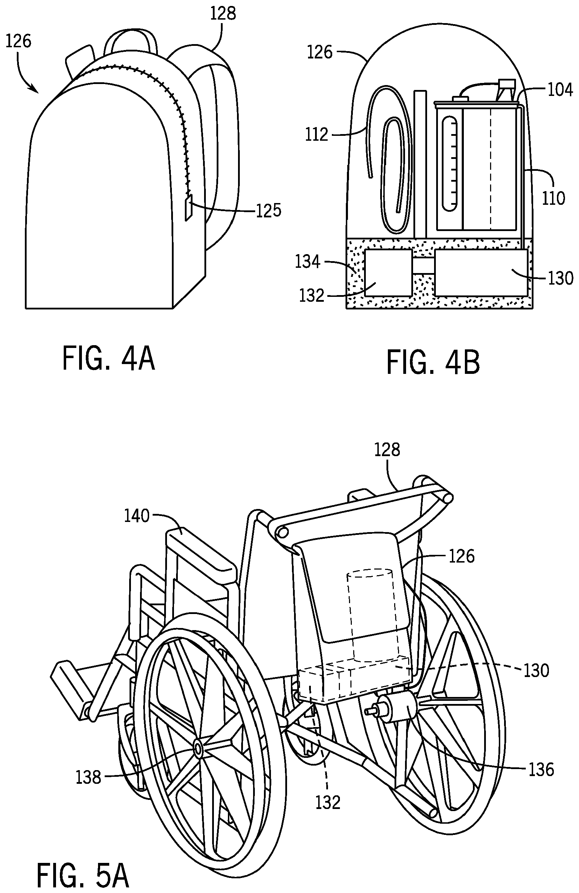

Referring now to **FIGS. 4A-4B**, the fluid collection system 100 may include a portable carrying case 126 that may be worn by a user and/or a caregiver. In some embodiments, the portable carrying case 126 may include one or more straps 128 to couple the portable carrying case 126 to at least one of the user, one or more wheelchair handles, a bed, or a table. In some embodiments, an outer surface of the portable carrying case 126 may include Velcro or other fasteners configured to couple directly to a wheelchair. The portable carrying case 126 may be a generally rectangular shape with a flat bottom for stability and for being able to fit underneath a wheelchair and/or coupled to a back of the wheelchair. The one or more straps 128 may be configured to be worn on a shoulder or to detachably secure, mount, or hang from a stationary or mobile structure (e.g., a wheelchair) and support the fluid collection system 100. In some embodiments, the portable carrying case 126 may include at least one of a backpack or shoulder bag. In some embodiments, the portable carrying case 126 may include a durable outer cover and/or shell.

In some embodiments, the portable carrying case 126 may include a fastening seam 125, such as a hook and loop, snap, or zipper seam. In some embodiments, the portable carrying case 126 may include more than one compartments, each compartment being able to be accessed by a fastening seam. Thus, the portable carrying case 126 may be closeable for discretion of the fluid collection system 100 and may be used to hold other items securely. For example, when the drain tube 112 is not being used, the drain tube 112 may be stored within the portable carrying case 126. The portable carrying case 126 may be sized and dimensioned to hold at least the fluid collection container 104, a pump 130, and a battery 132 (shown in **FIG. 4B**) therein.

The pump 130 may be put in fluid communication with the interior region of the fluid collection container 104 to pull at least a partial vacuum on the interior region of the fluid collection container 104 effective to draw the fluid from the fluid collection device 102 and into the fluid collection container 104. The pump 130 may be coupled directly to the fluid collection container 104, or the suction tube 110 may fluidly couple the pump 130 with the interior region of the fluid collection container 104.

The pump 130 includes a peristaltic pump and may include one or more of a manual vacuum pump, and electric vacuum pump, a diaphragm pump, a centrifugal pump, a displacement pump, a magnetically driven pump, or any pump configured to produce a vacuum. For example, the pump may include an air media diaphragm pump having a minimum pumping speed of 25 ml/second. In some embodiments, the pump 130 includes a variable speed pump and/or a continuous pump. For example, the pump 130 may include a variable speed pump. The pump 130 provides a vacuum or suction to remove fluid from the fluid collection device 102. In some examples, the pump 130 may be powered by one or more batteries 132 operatively coupled to the pump. In some embodiments, the battery 132 may include a lithium ion battery. In some embodiments, the battery 132 may be alkaline or rechargeable. In some examples, the pump 130 and/or the battery 132 may be sized and shaped to fit within the portable carrying case 126. For example, the pump 130 may include one or more miniaturized pumps or one or more micro pumps. The pump 130 may include one or more of a switch, a button, a plug, a remote, or any other device suitable to activate the pump 130.

As shown in **FIG 4B****,** the portable carrying case 126 may include a sound dampening material 134 where the pump 130 and/or the battery 132 may be located. In some embodiments, the sound dampening material 134 may be configured to dampen the operational sound of the pump 130 and secure the pump 130 and/or the battery 132, and/or other components within the portable carrying case 126. The sound dampening material 134 may include sound-deadening foam. Sound-deadening foam may include egg crate style foam constructed of open cell melamine, polyurethane, or other suitable foam material. The sound dampening material 134 may include a composite of synthetic or natural materials. In addition to abating sound, the sound dampening material 134 may minimize operational vibrations of the fluid collection system 100.

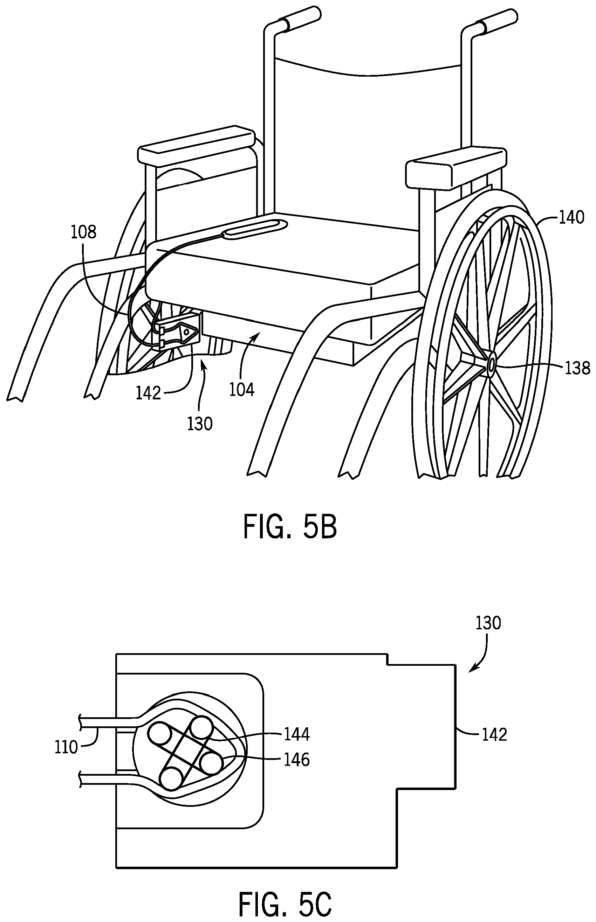

Referring now to **FIG. 5A****,** in some embodiments, the fluid collection system 100 may be powered by the battery 132 and may also include an additional power supply. The additional power supply may be used when the battery 132 is at a low power status or charging. In some embodiments, the additional power supply may include an outlet on an electric powered wheelchair. Thus, the fluid collection system 100 may couple to a receptacle on the electric powered wheelchair. In some embodiments, the fluid collection system 100 may include any suitable connector to electrically couple the fluid collection system 100 to the additional power supply (*e.g.,* USB port, Neutrik connector, XLR connector, A/C D/C converter, 3-pin charge socket, etc.).

In some embodiments, the fluid collection system 100 may include an alternator 136 to charge the battery 132 or power an electric motor for the pump 130. In some embodiments, the alternator 136 may be coupled to an axle 138 of the wheelchair 140. When the wheelchair is moved, the alternator 136 may be coupled to the wheel to generate a current that may charge the battery 132 and/or power the pump 130. The alternator 136 may extend the life of the battery 132 in operation by powering the components of the fluid collection system 100 (e.g. the pump 130) directly when the wheelchair 140 is moving. In some embodiments, the alternator 136 may charge the battery 132 and operate the pump 130 or other component of the fluid collection system 100 simultaneously.

Referring now to **FIGS. 5B-5C**, the pump 130 is powered directly by the motion of the wheelchair 140. The pump 130 includes a peristaltic pump 142. The peristaltic pump 142 includes a roller portion 144 configured to compress a tube (e.g., collection tube 108 or suction tube 110) to draw fluid from the fluid collection device 102 to the fluid collection container 104. In **FIG. 5B****,** the fluid collection container 104 is shown underneath a seat of the wheelchair 140. The roller portion 144 of the peristaltic pump 142 may be coupled directly to the axle 138 of the wheelchair 140. For example, when the wheelchair 140 moves forward the rotating axle 138 of the wheelchair 140 rotates the roller portion 144 of the peristaltic pump 142. In some embodiments, the roller portion 144 may be directly coupled to the axle 138. In other embodiments, the roller portion 144 may be coupled to a gear system (not shown) that establishes a gear ratio between the axle 138 and the roller portion 144 of the peristaltic pump 142. In some embodiments, the roller portion 144 may operate on a ratchet gear, such that when the wheelchair wheels, roll backwards, the roller portion 144 does not rotate in a reverse direction in order to prevent urine from flowing back to the fluid collection device 102.

The peristaltic pump 142 may include a second roller portion 146, in some embodiments. The roller portion 144 discussed above may be mechanically coupled to the axle 138 and the second roller portion 146 may be electrically coupled to the battery 132 or an alternate power supply discussed above. Thus, in some embodiments, the roller portion 144 of the pump 142 may be configured to receive energy from the mechanical motion of the wheel chair 140 to pump fluid through the peristaltic pump 142 and the second roller portion 146 may be configured to receive electrical energy from the battery 132 or alternate electrical power supply to pump fluid. In some embodiments, the roller portions 144 and 146 are configured to operate independently and/or synchronously. For example, when the wheelchair 140 is moving forward, the pump 130 may operate solely with the roller portion 144 to transfer fluid. When the wheelchair 140 is stopped or moving backwards, the pump 130 may operate only the second roller portion 146, and be powered by the battery 132, alternator 136, or other power supply.

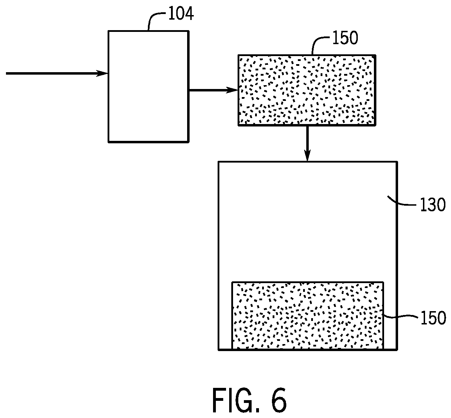

Referring now to **FIG. 6**, in some embodiments, the fluid collection system 100 may include one or more odor filters 150 configured to at least partially filter or neutralize an odor of the fluid. The filter 150 may be configured to neutralize odor of the air being pulled from the interior region of the fluid collection container 104 by the pump 130. In some embodiments, the filter 150 may be positioned between at least a portion of the collection tube 108 and a portion of the interior region of the fluid collection container 104 such that air being pulled from the interior region of the fluid collection container 104 may be filtered before or as the air enters the suction tube 110. In some embodiments, the filter 150 may be positioned on an exhaust vent on the pump 130. In some embodiments, a filter 150 may be positioned both at the exhaust vent on the pump 130 and before air enters the suction tube 110. The filter 150 may include an odor-absorbing filter and/or a hydrophobic filter configured to prevent or minimize fluid from the fluid collection container 104 being pulled into the pump 130.

In some embodiments, the filter 150 may include an activated carbon filter. In other embodiments, the filter 150 may include a gas filter. In some embodiments, the one or more odor filter 150 may be included on or within at least one of the fluid collection device 102, the portable carrying case 126, or the fluid collection container 104.

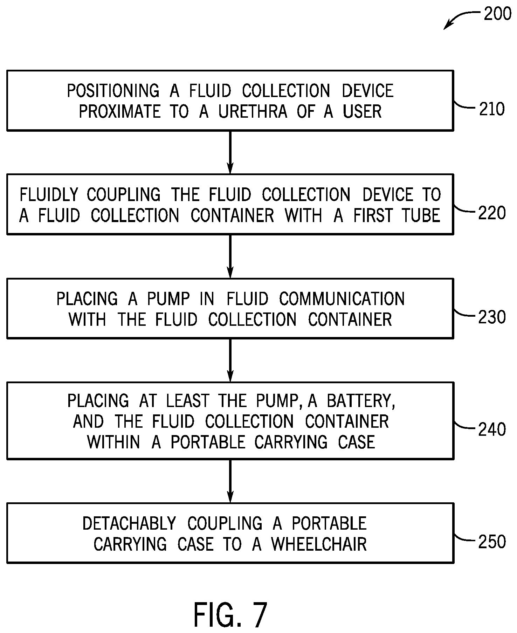

**FIG.** 7 is a flow diagram of a method 200 for assembling a portable fluid collection system. The method 200 includes an act 210 of positioning a fluid collection device proximate to a urethra of a user and an act 220 of fluidly coupling the fluid collection device to a fluid collection container with a first tube. The method 200 also includes an act 230 of placing a pump in fluid communication with the fluid collection container. The pump is configured to pull a vacuum on the interior region of the fluid collection container effective to draw fluid from the fluid collection device through the first tube into the fluid collection container. The method 200 may also include an act 240 of placing at least the pump, a battery, and the fluid collection container within a portable carrying case and an act 250 of detachably coupling a portable carrying case to a wheelchair.

The method 200 may include assembling any of the fluid collection system embodiments described herein. For example, the act 250 of detachably coupling a portable carrying case to a wheelchair may include coupling one or more straps of the portable carrying case to one or more handles of the wheelchair. The fluid collection container may include either a reusable container or a disposable container. The method of assembly the portable fluid collection system may include cleaning and replacing the container or disposing of the container and fluidly coupling a new fluid collection container to the fluid collection device. The method of assembly the portable fluid collection system may include energizing the pump with an alternation operatively coupled to an axle of the wheelchair.

The acts of the method of collecting fluids from a user described above are for illustrative purposes. For example, the acts of the method of collecting fluids from a user can be performed in different orders, split into multiple acts, modified, supplemented, or combined. One or more of the acts of the method of collecting fluids from a user can be omitted from the method. Any of the acts of the method of collecting fluids from a user can include using any of the portable fluid collection systems disclosed herein.

As used herein, the term "about" or "substantially" refers to an allowable variance of the term modified by "about" or "substantially" by ±10% or ±5%. Further, the terms "less than," "or less," "greater than," "more than," or "or more" include, as an endpoint, the value that is modified by the terms "less than," "or less," "greater than," "more than," or "or more."

## Claims

1. A fluid collection system (10), comprising:
a fluid collection device (12) configured to collect fluid discharged from the urethra of a user;
a fluid collection container (14) configured to receive the fluid from the fluid collection device; and
a pump (16) in fluid communication with the fluid collection container configured to pull an at least partial vacuum on an interior region of the fluid collection container effective to draw fluid from a fluid collection device into the fluid collection container; wherein the fluid collection container is either a reusable container or a disposable container, and wherein at least the pump and the disposable container are sized and dimensioned to be disposed within a portable carrying case;
wherein the pump includes a peristaltic pump, and the peristaltic pump includes a roller portion (144) configured to compress a tube, wherein the roller portion is configured to receive energy from the mechanical motion of a wheelchair (140) by being directly or indirectly coupled to the rotating axle of the wheelchair to pump fluid through the peristaltic pump (142).

2. The fluid collection system of claim 1, wherein the portable carrying case includes a sound dampening material, wherein at least the pump is disposed within the sound dampening material.

3. The fluid collection system of claim 2, wherein the sound dampening material includes a foam material configured to dampen operational sound of at least the pump.

4. The fluid collection system of any one of the preceding claims, wherein the roller portion (144) is a first roller portion, coupled to a wheel axle (138) of a wheelchair (140).

5. The fluid collection system of claim 4, wherein the peristaltic pump includes a second roller portion (146) operatively coupled to an electric motor, wherein the first roller portion and the second roller portion are configured to rotate independently.

6. A fluid collection system as claimed in claim 4 or 5, including a gear system to which the roller portion is coupled, which gear system establishes a gear ratio between the axle (138) of the wheelchair and the roller portion of the peristaltic pump (142).

7. A fluid collection system as claimed in any one of the preceding claims, wherein the roller portion (144) operates on a ratchet gear such that when the wheelchair wheels roll backwards the roller portion does not rotate in a reverse direction in order to prevent urine from flowing back to the fluid collection device (102).

8. A fluid collection system as claimed in claim 5 or claim 6 or 7 as dependent on claim 5, wherein the second roller portion (146) is configured to receive electrical energy from a battery or alternate electrical power supply to pump fluid.

9. A fluid collection system as claimed in claim 8, wherein the first (144) and second (146) roller portions are configured to operate independently and/or synchronously.

10. A fluid collection system, as claimed in any one of the preceding claims, which system is portable, and which includes:
a first tube (108) in fluid communication with the fluid collection device, wherein the
fluid collection container includes a rectangular shape, wherein the
pump is effective to draw fluid from the fluid collection device through the first tube into the fluid collection container; the portable fluid collection system having
a power supply (132) operatively coupled to the pump; and
a drainage tube (112) configured to couple to the fluid collection container.

11. The portable fluid collection system of claim 10, wherein the drainage tube (112) includes a valve (116) that opens to drain the fluid collection container.

12. The portable fluid collection system of claim 10, wherein the pump de-energizes when the drainage tube is coupled to the fluid collection container.

13. The portable fluid collection system of any of claims 10 to 12, wherein the portable fluid collection system further includes an alternator (136) coupled to a wheelchair wheel configured to charge the battery when the wheelchair wheel rotates.

## Patentansprüche

1. Flüssigkeitssammelsystem (10), umfassend:
eine Flüssigkeitssammelvorrichtung (12), die dazu ausgebildet ist, aus der Harnröhre eines Benutzers abgelassene Flüssigkeit zu sammeln;
einen Flüssigkeitssammelbehälter (14), der dazu ausgebildet ist, die Flüssigkeit aus der Flüssigkeitssammelvorrichtung aufzunehmen; und
eine Pumpe (16), die in Fluidverbindung mit dem Flüssigkeitssammelbehälter steht und dazu ausgebildet ist, in einem Innenbereich des Flüssigkeitssammelbehälters ein mindestens teilweises Vakuum zu erzeugen, das wirksam ist, um Flüssigkeit aus einer Flüssigkeitssammelvorrichtung in den Flüssigkeitssammelbehälter zu saugen; wobei der Flüssigkeitssammelbehälter entweder ein wiederverwendbarer Behälter oder ein Einwegbehälter ist, und wobei mindestens die Pumpe und der Einwegbehälter bemessen und dimensioniert sind, um innerhalb eines tragbaren Transportkoffers angeordnet zu werden;
wobei die Pumpe eine Peristaltikpumpe einschließt und die Peristaltikpumpe einen Rollenabschnitt (144) einschließt, der dazu ausgebildet ist, einen Schlauch zusammenzudrücken, wobei der Rollenabschnitt dazu ausgebildet ist, Energie aus der mechanischen Bewegung eines Rollstuhls (140) aufzunehmen, indem er direkt oder indirekt mit der Drehachse des Rollstuhls gekoppelt ist, um Flüssigkeit durch die Peristaltikpumpe (142) zu pumpen.

2. Flüssigkeitssammelsystem nach Anspruch 1, wobei der tragbare Transportkoffer ein schalldämpfendes Material einschließt, wobei mindestens die Pumpe innerhalb des schalldämpfenden Materials angeordnet ist.

3. Flüssigkeitssammelsystem nach Anspruch 2, wobei das schalldämpfende Material ein Schaumstoffmaterial einschließt, das dazu ausgebildet ist, Betriebsgeräusche von mindestens der Pumpe zu dämpfen.

4. Flüssigkeitssammelsystem nach einem der vorstehenden Ansprüche, wobei der Rollenabschnitt (144) ein erster Rollenabschnitt ist, der mit einer Radachse (138) eines Rollstuhls (140) gekoppelt ist.

5. Flüssigkeitssammelsystem nach Anspruch 4, wobei die Peristaltikpumpe einen zweiten Rollenabschnitt (146) einschließt, der mit einem Elektromotor wirkverbunden ist, wobei der erste Rollenabschnitt und der zweite Rollenabschnitt dazu ausgebildet sind, sich unabhängig voneinander zu drehen.

6. Flüssigkeitssammelsystem nach Anspruch 4 oder 5, einschließlich eines Getriebesystems, mit dem der Rollenabschnitt gekoppelt ist, wobei das Getriebesystem zwischen der Achse (138) des Rollstuhls und dem Rollenabschnitt der Peristaltikpumpe (142) ein Übersetzungsverhältnis herstellt.

7. Flüssigkeitssammelsystem nach einem der vorstehenden Ansprüche, wobei der Rollenabschnitt (144) mit einem Sperrklinkenritzel zusammenwirkt, sodass sich der Rollenabschnitt nicht in umgekehrter Richtung dreht, wenn die Rollstuhlräder rückwärts rollen, um zu verhindern, dass Urin in die Flüssigkeitssammelvorrichtung (102) zurückfließt.

8. Flüssigkeitssammelsystem nach Anspruch 5 oder 6 oder 7, wenn abhängig von Anspruch 5, wobei der zweite Rollenabschnitt (146) dazu ausgebildet ist, elektrische Energie von einer Batterie oder einer alternativen Stromversorgung aufzunehmen, um Flüssigkeit zu pumpen.

9. Flüssigkeitssammelsystem nach Anspruch 8, wobei der erste (144) und der zweite (146) Rollenabschnitt dazu ausgebildet sind, unabhängig voneinander und/oder synchron zu arbeiten.

10. Flüssigkeitssammelsystem nach einem der vorstehenden Ansprüche, wobei das System tragbar ist und einschließt:
einen ersten Schlauch (108), der in Fluidverbindung mit der Flüssigkeitssammelvorrichtung steht, wobei der
Flüssigkeitssammelbehälter eine rechteckige Form einschließt, wobei die
Pumpe wirksam ist, um Flüssigkeit aus der Flüssigkeitssammelvorrichtung durch den ersten Schlauch in den Flüssigkeitssammelbehälter zu saugen; wobei das tragbare Flüssigkeitssammelsystem Folgendes aufweist
eine Stromversorgung (132), die mit der Pumpe wirkverbunden ist; und
einen Ablassschlauch (112), der zur Kopplung mit dem Flüssigkeitssammelbehälter ausgebildet ist.

11. Tragbares Flüssigkeitssammelsystem nach Anspruch 10, wobei der Ablassschlauch (112) ein Ventil (116) einschließt, das sich öffnet, um den Flüssigkeitssammelbehälter zu entleeren.

12. Tragbares Flüssigkeitssammelsystem nach Anspruch 10, wobei die Pumpe sich abschaltet, wenn der Ablassschlauch mit dem Flüssigkeitssammelbehälter gekoppelt ist.

13. Tragbares Flüssigkeitssammelsystem nach einem der Ansprüche 10 bis 12, wobei das tragbare Flüssigkeitssammelsystem weiter einen Wechselstromgenerator (136) einschließt, der mit einem Rollstuhlrad gekoppelt ist und dazu ausgebildet ist, die Batterie aufzuladen, wenn sich das Rollstuhlrad dreht.

## Revendications

1. Système (10) de collecte de fluide, comprenant :
un dispositif (12) de collecte de fluide configuré pour collecter un fluide évacué de l'urètre d'un utilisateur ;
un récipient (14) de collecte de fluide configuré pour recevoir le fluide provenant du dispositif de collecte de fluide ; et
une pompe (16) en communication fluide avec le récipient de collecte de fluide configurée pour créer un vide au moins partiel sur une région intérieure du récipient de collecte de fluide efficace pour aspirer du fluide à partir d'un dispositif de collecte de fluide dans le récipient de collecte de fluide ; dans lequel le récipient de collecte de fluide est soit un récipient réutilisable, soit un récipient jetable, et dans lequel au moins la pompe et le récipient jetable sont d'une taille et d'une dimension leur permettant d'être disposés dans un boîtier de transport portatif ;
dans lequel la pompe inclut une pompe péristaltique, et la pompe péristaltique inclut une partie de rouleau (144) configurée pour comprimer un tube, dans lequel la partie de rouleau est configurée pour recevoir de l'énergie du mouvement mécanique d'un fauteuil roulant (140) en étant couplée directement ou indirectement à l'axe rotatif du fauteuil roulant pour pomper du fluide à travers la pompe péristaltique (142).

2. Système de collecte de fluide selon la revendication 1, dans lequel le boîtier de transport portatif inclut un matériau d'insonorisation, dans lequel au moins la pompe est disposée à l'intérieur du matériau d'insonorisation.

3. Système de collecte de fluide selon la revendication 2, dans lequel le matériau d'insonorisation inclut un matériau en mousse configuré pour atténuer le bruit de fonctionnement d'au moins la pompe.

4. Système de collecte de fluide selon l'une quelconque des revendications précédentes, dans lequel la partie de rouleau (144) est une première partie de rouleau, couplée à un axe (138) de roue d'un fauteuil roulant (140).

5. Système de collecte de fluide selon la revendication 4, dans lequel la pompe péristaltique inclut une deuxième partie de rouleau (146) couplée fonctionnellement à un moteur électrique, dans lequel la première partie de rouleau et la deuxième partie de rouleau sont configurées pour tourner indépendamment.

6. Système de collecte de fluide selon la revendication 4 ou la revendication 5, incluant un système d'engrenage auquel la partie de rouleau est couplée, lequel système d'engrenage établit un rapport d'engrenage entre l'axe (138) du fauteuil roulant et la partie de rouleau de la pompe péristaltique (142).

7. Système de collecte de fluide selon l'une quelconque des revendications précédentes, dans lequel la partie de rouleau (144) fonctionne sur un engrenage à cliquet de sorte que lorsque les roues du fauteuil roulant roulent vers l'arrière, la partie de rouleau ne tourne pas dans une direction inverse afin d'empêcher de l'urine de refluer vers le dispositif de collecte (102) de fluide.

8. Système de collecte de fluide selon la revendication 5 ou la revendication 6 ou la revendication 7 lorsqu'elle dépend de la revendication 5, dans lequel la deuxième partie de rouleau (146) est configurée pour recevoir de l'énergie électrique provenant d'une batterie ou d'une alimentation électrique alternative pour pomper du fluide.

9. Système de collecte de fluide selon la revendication 8, dans lequel les première (144) et deuxième (146) parties de rouleau sont configurées pour fonctionner indépendamment et/ou de manière synchrone.

10. Système de collecte de fluide selon l'une quelconque des revendications précédentes, lequel système est portatif, et qui inclut :
un premier tube (108) en communication fluide avec le dispositif de collecte de fluide, dans lequel le récipient de collecte de fluide inclut une forme rectangulaire, dans lequel la
pompe est efficace pour aspirer le fluide à partir du dispositif de collecte de fluide à travers le premier tube dans le récipient de collecte de fluide ; le système de collecte de fluide portatif présentant
une alimentation électrique (132) couplée fonctionnellement à la pompe ; et
un tube de drainage (112) configuré pour être couplé au récipient de collecte de fluide.

11. Système de collecte de fluide portatif selon la revendication 10, dans lequel le tube de drainage (112) inclut une vanne (116) qui s'ouvre pour drainer le récipient de collecte de fluide.

12. Système de collecte de fluide portatif selon la revendication 10, dans lequel la pompe se met hors tension lorsque le tube de drainage est couplé au récipient de collecte de fluide.

13. Système de collecte de fluide portatif selon l'une quelconque des revendications 10 à 12, dans lequel le système de collecte de fluide portatif inclut en outre un alternateur (136) couplé à une roue de fauteuil roulant configurée pour charger la batterie lorsque la roue de fauteuil roulant tourne.
